# EUROPEAN PATENT SPECIFICATION

(11) **EP 4 467 110 B1**
(45) Date of publication and mention of the grant of the patent: **17.12.2025**
(21) Application number: 24171102.7
(22) Date of filing: 18.04.2024
(51) Int. Cl.: A61F 2/64, A61F 2/74, A61F 2/68

(54) **PROSTHETIC KNEE JOINT**
KNIEGELENKPROTHESE
PROTHÈSE DE GENOU

(30) Priority: 22.05.2023 TW 112118862
(43) Date of publication of application: 27.11.2024
(73) Proprietor: PRO LIMB International Corp., New Taipei City (TW)
(72) Inventor: Shen, Hsin-Fa, New Taipei City (TW)
(74) Representative: Petraz, Gilberto Luigi

(56) References cited:
- US-A1- 2020 323 655
- US-B1- 6 706 074

## Description

### 1. Field of the Invention

The present invention relates to a prosthesis, especially to a modular knee joint. Document US 2020/323655 A1 discloses the features of the preamble of claim 1.

### 2. Description of the Prior Arts

The traditional modular knee joints are single-shaft-bearing-and-actuating devices and are assembled between a thigh and a prosthetic shank of a user. When the user put the weight on the prosthetic shank and the modular knee joint, the modular knee joint activates the resistance mechanism and selectively provides the supporting force for bending. Therefore, the modular knee joint can support the user's weight, let the modular knee joint bend slowly, and facilitate the user to stand or walk by feet.

During walking, the user steps forward with the normal foot and move the center of gravity to the body on the front side. The thigh with the prosthesis on the back side pushes down against the prosthetic shank, causing the toes of the prosthesis to receive reaction force from the ground. A moment is generated on the prosthetic shank, causing the modular knee joint to bend. When the single-shaft mechanism in the traditional modular knee joint deflects due to the torque, the resistance mechanism is turned off, allowing the modular knee joint to bend significantly without resistance. Then the user swings the thigh with the prosthesis on the back side forward and thus the modular knee joint turns straight again. After the heel of the prosthesis receives reaction force from the ground and pushes against the modular knee joint, the resistance mechanism is turned on again. The resistance mechanism allows the prosthesis and the modular knee joint to support the user's weight, so the user can take the next step.

To keep the user's steps at an appropriate pace, when the thigh with the prosthesis is at the back side, the toes press down and generate a moment on the modular knee joint. The threshold of turning off the resistance mechanism must be as sensitive as possible to avoid the user's waiting for the resistance mechanism on the modular knee joint to be turned off between stepping out the normal foot and then stepping out the prosthesis. If the process of turning off the resistance mechanism is not sensitive and swift enough, the user will have an obvious stumbling feeling during walking, which will affect the pace.

However, since the traditional prosthetic knee joints are single-shaft-bearing-and-actuating device, when the body's weight applies pressure on the prosthetic shank, the heel or the toes of the prosthetic shank may activate the actuating device, and thus cause the prosthetic shank to stumble when it steps out. Therefore, it is necessary to install an elastic adjustment assembly as an anti-actuating device to reduce the sensitivity of the actuating device and the stumbling feeling when the prosthetic shank steps out. However, the elastic adjustment assembly will also reduce the sensitivity of the actuating device, resulting in incomplete startup or loss of resistance of the actuating device, which affects safety.

To overcome the shortcomings, the present invention provides a modular knee joint to mitigate or obviate the aforementioned problems.

The main objective of the present invention is to provide a modular knee joint that is configured to connect a thigh and a prosthetic shank of a user which allows the user to go down the stairs or walk on uneven ground.

The modular knee joint has an upper connecting unit, a lower connecting unit, a connecting rod assembly, a locking assembly, and an actuating assembly. The upper connecting unit is configured to connect the thigh of the user. The lower connecting unit is configured to connect the prosthetic shank and be securely mounted on the actuating assembly. The connecting rod assembly is located between the upper connecting unit and the lower connecting unit. The locking assembly selectively locks the connecting rod assembly; therefore, the connecting rod assembly is incapable of pivoting.

The actuating assembly has a first oil storage cavity, a second oil storage cavity, a first connecting channel, a second connecting channel, a movable plug, and a driven unit. The first connecting channel fluidly communicates with the first oil storage cavity and the second oil storage cavity, and the second connecting channel also fluidly communicates with the first oil storage cavity and the second oil storage cavity. The movable plug selectively blocks the first connecting channel. An end of the driven unit is connected to the connecting rod assembly, and another end of the driven unit selectively pushes the movable plug.

Wherein when the upper connecting unit and the lower connecting unit rotate with respect to each other, the driven unit and the movable plug are separated by the connecting rod assembly. Then the movable plug blocks the first connecting channel, and the locking assembly locks the connecting rod assembly at the same time. Therefore, the connecting rod assembly is incapable of pivoting, and the driven unit and the movable plug keep separated.

Through the present invention, the bending process of the modular knee joint can be maintained at a slow bending. When the user wears the modular knee joint and goes down the stairs, the bending angle of the modular knee joint can be maintained when the foot with the modular knee joint is behind, such that the modular knee joint will not lose resistance and the user will not fall on the stairs. Similarly, the user can also walk smoothly when walking on uneven ground with height differences. Therefore, if the heel contacts the ground during walking, the resistance mechanism of the modular knee joint is turned on during the full-angle bending process and the modular knee joint can be maintained at a slow bending. In contrast, the resistance mechanism of the modular knee joint is turned off when the toes contact the ground during walking.

Other objectives, advantages and novel features of the invention will become more apparent from the following detailed description when taken in conjunction with the accompanying drawings.

### IN THE DRAWINGS

Fig. 1 is a perspective view of a modular knee joint in accordance with the present invention;
Fig. 2 is another perspective view of the modular knee joint in Fig. 1;
Fig. 3 is a side view of the modular knee joint in Fig. 1;
Fig. 4 is a front view of the modular knee joint in Fig. 1;
Fig. 5 is a sectional view of the modular knee joint across line 5-5 in Fig. 4;
Fig. 6 is an enlarged view of the modular knee joint in Fig. 5;
Fig. 7 is a sectional view of the modular knee joint across line 7-7 in Fig. 4;
Fig. 8 is a sectional view of the modular knee joint after bending across line 5-5 in Fig. 4; and
Fig. 9 is an enlarged view of the modular knee joint after bending in Fig. 8.

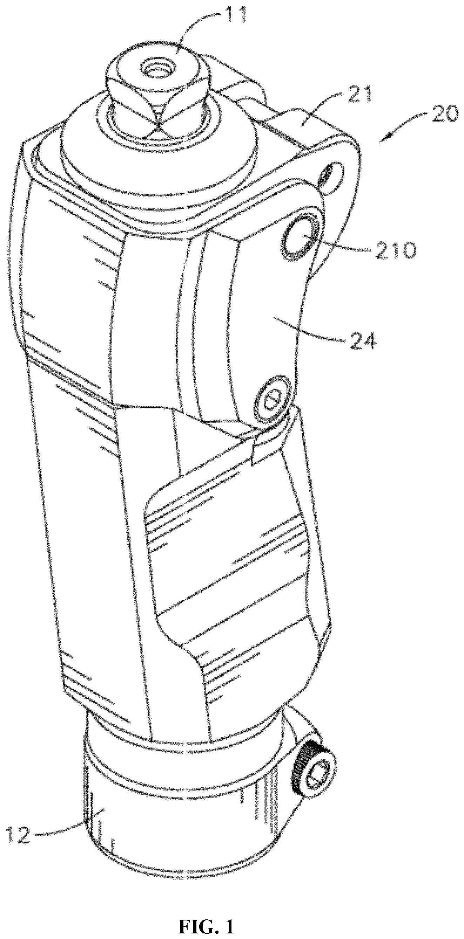

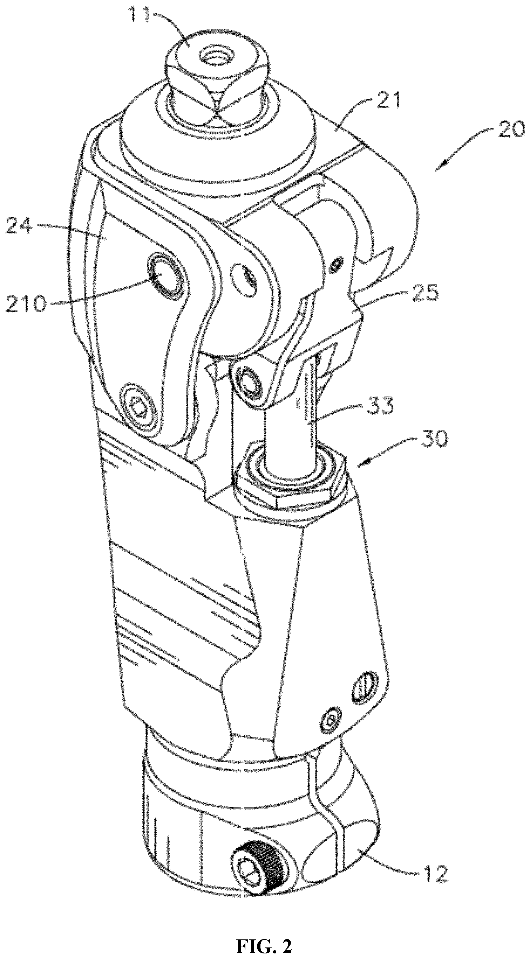

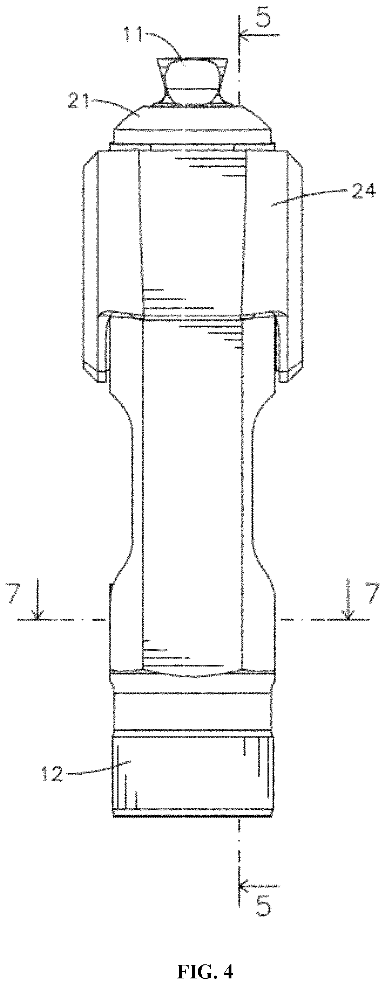

With reference to Fig. 1 to Fig. 4 first, a modular knee joint in accordance with the present invention has buffering effect and restriction effect and is configured to connect a thigh and a prosthetic shank of a user. The modular knee joint comprises an upper connecting unit 11, a lower connecting unit 12, a connecting rod assembly 20, an actuating assembly 30, and a locking assembly 40.

The upper connecting unit 11 is configured to connect the thigh of the user. The lower connecting unit 12 is configured to connect the prosthetic shank. The connecting rod assembly 20, the locking assembly 40, and the actuating assembly 30 are mounted between the upper connecting unit 11 and the lower connecting unit 12.

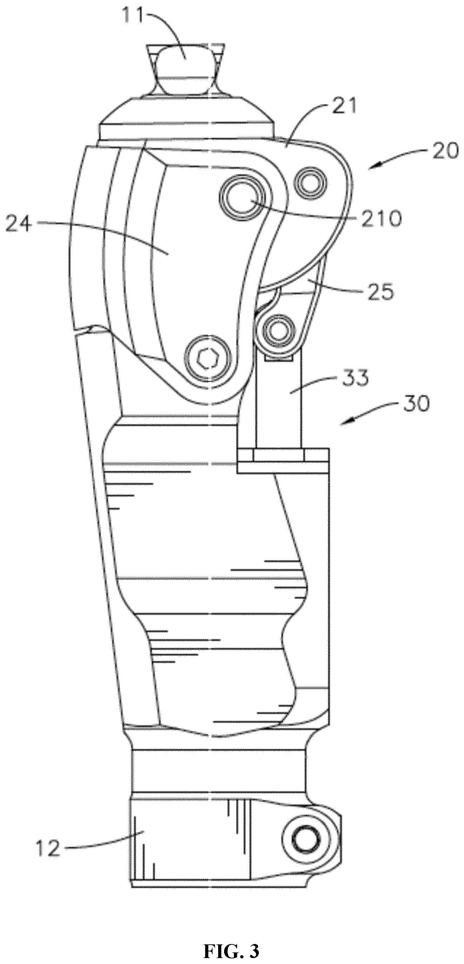

With reference to Fig. 3 to Fig. 5, the connecting rod assembly 20 comprises a main connecting rod 21, an auxiliary connecting rod 22, a first connecting rod 23, a second connecting rod 24, and a third connecting rod 25. Wherein the main connecting rod 21, the auxiliary connecting rod 22, the first connecting rod 23, and the second connecting rod 24 form a four-bar linkage and can simulate subtle changes in the actual bending of the human knee joint. A third connecting rod 25 connects the four-bar linkage and the actuating assembly 30. The upper connecting unit 11 is securely mounted on the main connecting rod 21. The main connecting rod 21 comprises a front side, a back side, and a main pivot shaft 210. The main pivot shaft 210 is located between the front side and the back side of the main connecting rod 21. An end of the auxiliary connecting rod 22 is pivotably mounted on the main pivot shaft 210 of the main connecting rod 21.

The first connecting rod 23 comprises a first upper end and a first lower end. The first upper end is connected to the main connecting rod 21 and the first lower end is pivotably mounted on the actuating assembly 30. Precisely, the first upper end of the first connecting rod 23 is pivotably mounted on an end opposite to the main pivot shaft 210 of the auxiliary connecting rod 22. The second connecting rod 24 comprises a second upper end and a second lower end. The second upper end is connected to the main pivot shaft 210 of the main connecting rod 21 and the second lower end is pivotably mounted on the actuating assembly 30. The third connecting rod 25 comprises a third upper end and a third lower end. The third upper end is pivoted on the back side of the main connecting rod 21 and the third lower end is connected to the actuating assembly 30.

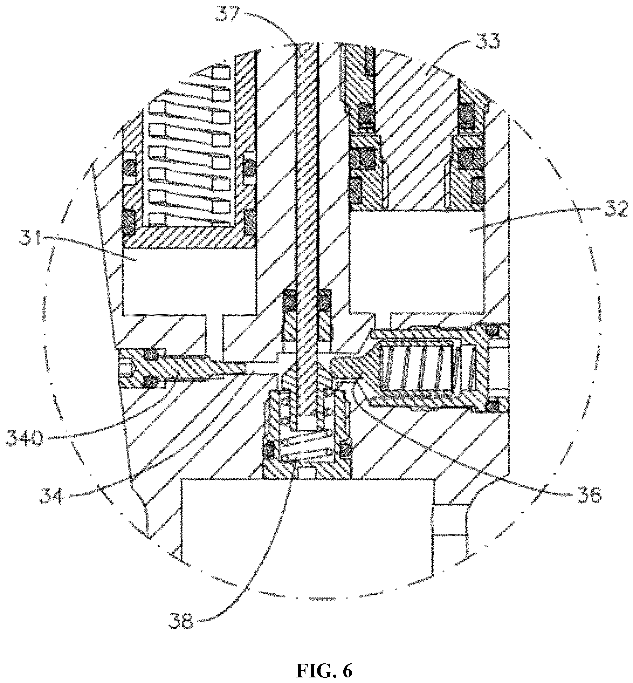

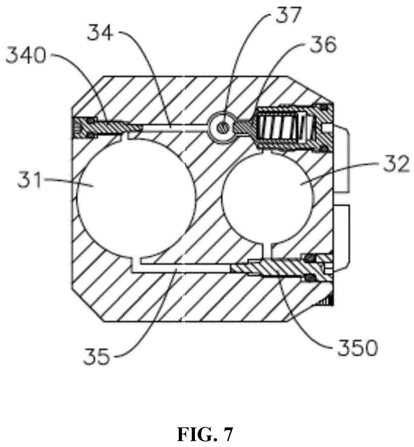

With reference to Fig. 6 and Fig. 7, the lower connecting unit 12 is securely mounted on the actuating assembly 30. The actuating assembly 30 comprises a first oil storage cavity 31, a second oil storage cavity 32, a plunger shaft 33, a first connecting channel 34, a second connecting channel 35, a movable plug 36, and a driven unit 37. In this embodiment, the actuating assembly 30 also comprises a first regulating plug 340 and a second regulating plug 350. The plunger shaft 33 is mounted through the second oil storage cavity 32 and is pivoted on the third lower end of the third connecting rod 25. The first connecting channel 34 and the second connecting channel 35 fluidly communicate with the first oil storage cavity 31 and the second oil storage cavity 32. The first regulating plug 340 is mounted through the first connecting channel 34 and can control a depth of the first regulating plug 340 penetrating into the first connecting channel 34. The first regulating plug 340 comprises a conical surface. By controlling the depth of the first regulating plug 340, a gap between the conical surface and an inner wall of the first connecting channel 34 can be adjusted, so the flow rate of hydraulic oil flowing between the first oil storage cavity 31 and the second oil storage cavity 32 can be adjusted. Similarly, the second regulating plug 350 is mounted through the second connecting channel 35 and can control a depth of the second regulating plug 350 penetrating into the second connecting channel 35. The second regulating plug 350 comprises a conical surface. By controlling the depth of the second regulating plug 350, a gap between the conical surface and an inner wall of the second connecting channel 35 can be adjusted, so the flow rate of hydraulic oil flowing between the first oil storage cavity 31 and the second oil storage cavity 32 can be adjusted.

The movable plug 36 selectively blocks the first connecting channel 34. An end of the driven unit 37 is connected to the connecting rod assembly 20 and another end of the driven unit 37 is selectively pushed against the movable plug 36. Precisely, the end of the driven unit 37 is connected to the first connecting rod 23 and another end of the driven unit 37 comprises an inclined plane. The inclined plane of the driven unit 37 is selectively pushed against the movable plug 36. In this embodiment, the actuating assembly 30 further comprises a driven elastic unit 38. The driven elastic unit 38 is connected to the driven unit 37 and tends to push the driven unit 37 against the movable plug 36.

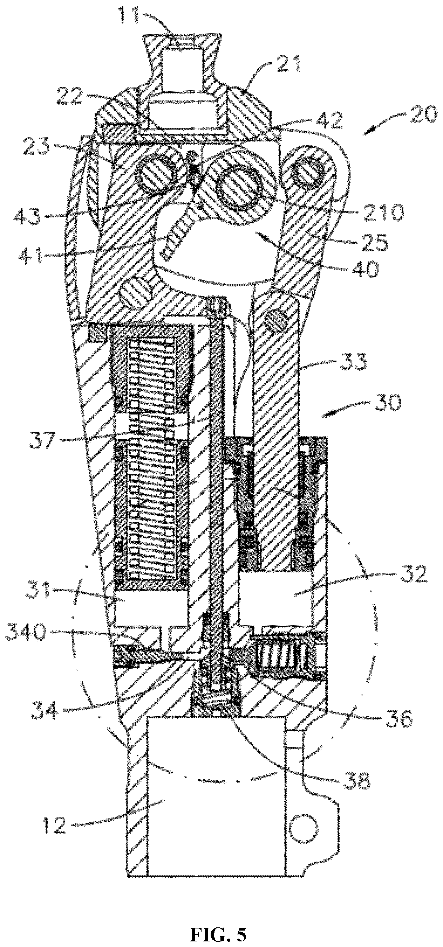

With reference to Fig. 5 again, the locking assembly 40 selectively locks the connecting rod assembly 20. Therefore, the connecting rod assembly 20 cannot be pivoted. Precisely, the locking assembly 40 comprises a locking unit 41, an elastic unit 42, and a limiting unit 43. An end of the locking unit 41 is connected to the main connecting rod 21 and another end of the locking unit 41 is selectively pushed against the first connecting rod 23. An end of the elastic unit 42 is connected to the auxiliary connecting rod 22. Another end of the elastic unit 42 is connected to the locking unit 41 and tends to push the locking unit 41 against the first connecting rod 23. The limiting unit 43 is mounted on the main connecting rod 21 and located between the locking unit 41 and the first connecting rod 23.

With reference to Fig. 5 and Fig. 6, when the user's leg is straightened, that is, when the thigh and the prosthetic shank remain in a straight line, the upper connecting unit 11 and the lower connecting unit 12 also remain in a straight line. At this time, the limiting unit 43 blocks between the locking unit 41 and the first connecting rod 23, so the locking unit 41 and the first connecting rod 23 are not in contact. At the same time, the driven unit 37 is affected by the traction and the elastic force of the linked elasticity of the first connecting rod 23, and the inclined plane of the driven unit 37 abuts the movable plug 36, so the movable plug 36 cannot block the first connecting channel 34. When the first connecting channel 34 remains connected, the hydraulic oil in the second oil storage cavity 32 can easily flow into the second oil storage cavity 32 through the first connecting channel 34. In other words, the plunger shaft 33 can move freely within the second oil storage cavity 32 to change an internal volume of the second oil storage cavity 32, such that the user can easily bend the modular knee joint.

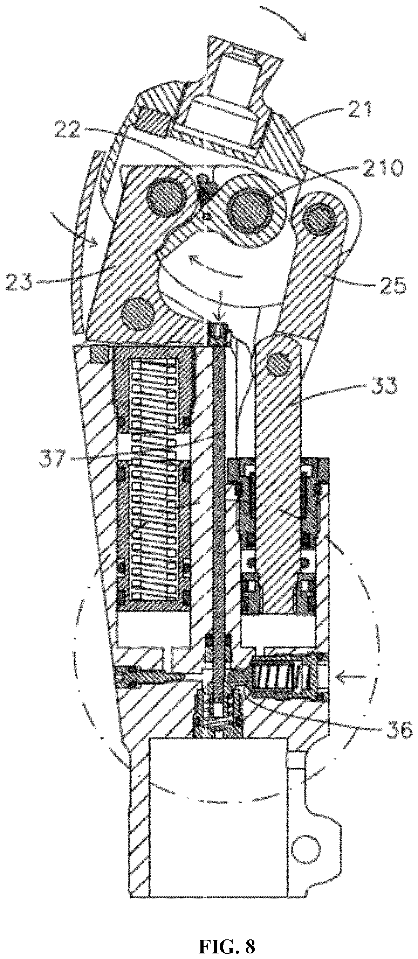

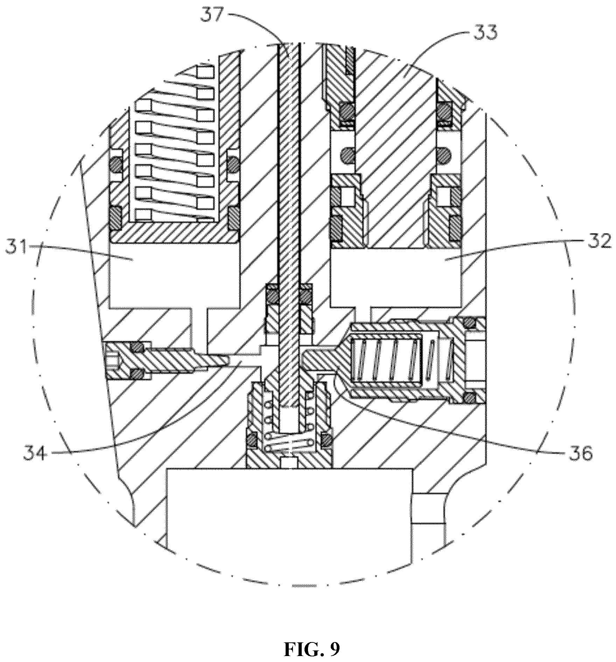

With reference to Fig. 8 and Fig. 9, when the user's leg is bended, the upper connecting unit 11 and the lower connecting unit 12 relatively rotate to an actuating angle, and the driven unit 37 and the movable plug 36 are separated by the connecting rod assembly 20. Precisely, when the upper connecting unit 11 and the lower connecting unit 12 relatively rotate to the actuating angle, the first connecting rod 23 rotates to engage with the locking unit 41 at the same time, such that the driven unit 37 keeps separating from the movable plug 36. When the first connecting rod 23 and the locking unit 41 engage with each other, the locking unit 41 is pushed against the first connecting rod 23, thereby preventing the first connecting rod 23 from continuing to rotate relatively to the main connecting rod 21. In other words, as long as the user bends the modular knee joint, no matter what the angle is, the locking unit 41 will engage with the first connecting rod 23.

When the driven unit 37 and the movable plug 36 are separated, the movable plug 36 can block the first connecting channel 34. At this time, the hydraulic oil can only flow from the second oil storage cavity 32 to the first oil storage cavity 31 through the second connecting channel 35. Since the gap of the second connecting channel 35 is small, the flow rate of the hydraulic oil through the second connecting channel 35 is very slow, such that the plunger shaft 33 cannot easily penetrate the second oil storage cavity 32, causing a resistance that prevents the user from further smooth bending. In other words, at this time, the connecting rod assembly 20 cannot pivot further, and the user's modular knee joint can maintain a slow bend.

After maintaining this posture for a period time, a certain amount of hydraulic oil can still flow to the first oil storage cavity 31 through the second oil storage cavity 32, allowing the plunger shaft 33 to penetrate the second oil storage cavity 32, so the bending angle of the user's modular knee joint can still slowly increase. That is, the user can keep the modular knee joint bending with the resistance during the entire bending process of the modular knee joint, and the modular knee joint will not lose the resistance after bending in a specific angle, so it can maintain the effect of gentle bending at all time.

Through the present invention, the bending process of the modular knee joint can be maintained at a slow bending. When the user wears the modular knee joint and goes down the stairs, the bending angle of the modular knee joint can be maintained when the foot with the modular knee joint is behind, such that the modular knee joint will not lose resistance and the user will not fall on the stairs. Similarly, the user can also walk smoothly when walking on uneven ground with height differences. Therefore, if the heel contacts the ground during walking, the resistance mechanism of the modular knee joint is turned on during the full-angle bending process and the modular knee joint can be maintained at a slow bending. In contrast, the resistance mechanism of the modular knee joint is turned off when the toes contact the ground during walking.

## Claims

1. A modular knee joint configured to connect a thigh and a prosthetic shank of a user and **characterized in that** the modular knee joint comprises:
an upper connecting unit (11) configured to connect the thigh of the user;
a lower connecting unit (12) configured to connect the prosthetic shank;
a connecting rod assembly (20) located between the upper connecting unit (11) and the lower connecting unit (12);
an actuating assembly (30), the lower connecting unit (12) securely mounted on the actuating assembly (30); the actuating assembly (30) comprising:
a first oil storage cavity (31);
a second oil storage cavity (32);
a first connecting channel (34) fluidly communicating with the first oil storage cavity (31) and the second oil storage cavity (32);
a second connecting channel (35) fluidly communicating with the first oil storage cavity (31) and the second oil storage cavity (32);
a movable plug (36) selectively blocking the first connecting channel (34); **characterized in that** the modular knee joint further comprises:
a locking assembly (40) selectively locking the connecting rod assembly (20), therefore the connecting rod assembly (20) incapable of pivoting; and
a driven unit (37), an end of the driven unit (37) connected to the connecting rod assembly (20), and another end of the driven unit (37) selectively pushing the movable plug (36);
wherein when the upper connecting unit (11) and the lower connecting unit (12) rotate with respect to each other, the driven unit (37) and the movable plug (36) are separated by the connecting rod assembly (20); then the movable plug (36) blocks the first connecting channel (34), and the locking assembly (40) locks the connecting rod assembly (20) at the same time; therefore, the connecting rod assembly (20) is incapable of pivoting and the driven unit (37) and the movable plug (36) keep separated.

2. The modular knee joint as claimed in claim 1, wherein the connecting rod assembly (20) comprises:
a main connecting rod (21), the upper connecting unit (11) securely mounted on the main connecting rod (21), the main connecting rod (21) comprising a main pivot shaft (210) located between a front side of the main connecting rod (21) and a back side of the main connecting rod (21);
a first connecting rod (23) comprising a first upper end and a first lower end, the first upper end connected to the main connecting rod (21), and the first lower end pivotably mounted on the actuating assembly (30); an end of the driven unit (37) connected to the first connecting rod (23);
a second connecting rod (24) comprising a second upper end and a second lower end, the second upper end connected to the main pivot shaft (210) of the main connecting rod (21), and the second lower end pivotably mounted on the actuating assembly (30); and
an auxiliary connecting rod (22), an end of the auxiliary connecting rod (22) pivotably mounted on the main pivot shaft (210) of the main connecting rod (21), and another end of the auxiliary connecting rod (22) pivotably mounted on the first upper end of the first connecting rod (23).

3. The modular knee joint as claimed in claim 2, wherein the locking assembly (40) comprises:
a locking unit (41), an end of the locking unit (41) connected to the main connecting rod (21), and another end of the locking unit (41) selectively pushing the first connecting rod (23);
wherein when the upper connecting unit (11) and the lower connecting unit (12) rotate with respect to each other, the locking unit (41) pushes the first connecting rod (23) and thereby prevents the first connecting rod (23) from rotating with respect to the main connecting rod (21).

4. The modular knee joint as claimed in claim 3, wherein, the locking assembly (40) comprises:
an elastic unit (42), an end of the elastic unit (42) connected to the auxiliary connecting rod (22), another end of the elastic unit (42) connected to the locking unit (41), and the elastic unit (42) configured to drive the locking unit (41) to push the first connecting rod (23).

5. The modular knee joint as claimed in claim 4, wherein the locking assembly (40) comprises:
a limiting unit (43) mounted on the main connecting rod (21) and located between the locking unit (41) and the first connecting rod (23).

6. The modular knee joint as claimed in claim 2, wherein the connecting rod assembly (20) comprises:
a third connecting rod (25) comprising a third upper end and a third lower end, and the third upper end pivoted to the backside of the main connecting rod (21).

7. The modular knee joint as claimed in claim 6, wherein the actuating assembly (30) comprises a plunger shaft (33) mounted through the second oil storage cavity (32) and connected to the third lower end of the third connecting rod (25).

8. The modular knee joint as any one of claimed from claim 1 to claim 7, wherein the driven unit (37) comprises an inclined plane selectively pushing the movable plug (36).

9. The modular knee joint as any one of claimed from claim 1 to claim 7, wherein the actuating assembly (30) comprises a driven elastic unit (42) connected to the driven unit (37) and configured to drive the driven unit (37) to push the movable plug (36).

## Patentansprüche

1. Modulares Kniegelenk, das dafür ausgelegt ist, einen Oberschenkel und einen prothetischen Unterschenkel eines Benutzers zu verbinden, und **dadurch gekennzeichnet, dass** das modulare Kniegelenk umfasst:
eine obere Verbindungseinheit (11), die dafür ausgelegt ist, den Oberschenkel des Benutzers zu verbinden;
eine untere Verbindungseinheit (12), die dafür ausgelegt ist, den prothetischen Unterschenkel zu verbinden;
eine Verbindungsstangenanordnung (20), die zwischen der oberen Verbindungseinheit (11) und der unteren Verbindungseinheit (12) gelegen ist;
eine Betätigungsanordnung (30), wobei die untere Verbindungseinheit (12) an der Betätigungsanordnung (30) fest montiert ist; wobei die Betätigungsanordnung (30) umfasst:
einen ersten Öllagerungshohlraum (31);
einen zweiten Öllagerungshohlraum (32);
einen ersten Verbindungskanal (34), der mit dem ersten Öllagerungshohlraum (31) und dem zweiten Öllagerungshohlraum (32) fluidmäßig kommuniziert;
einen zweiten Verbindungskanal (35), der mit dem ersten Öllagerungshohlraum (31) und dem zweiten Öllagerungshohlraum (32) fluidmäßig kommuniziert;
einen beweglichen Stopfen (36), der den ersten Verbindungskanal (34) selektiv sperrt;
**dadurch gekennzeichnet, dass** das modulare Kniegelenk ferner umfasst:
eine Verriegelungsanordnung (40), die die Verbindungsstangenanordnung (20) selektiv verriegelt, wodurch die Verbindungsstangenanordnung (20) unfähig wird, zu schwenken; und
eine angetriebene Einheit (37), wobei ein Ende der angetriebenen Einheit (37) mit der Verbindungsstangenanordnung (20) verbunden ist, und ein anderes Ende der angetriebenen Einheit (37) den beweglichen Stopfen (36) selektiv drückt;
worin, wenn die obere Verbindungseinheit (11) und die untere Verbindungseinheit (12) sich relativ zueinander drehen, die angetriebene Einheit (37) und der bewegliche Stopfen (36) von der Verbindungsstangenanordnung (20) getrennt sind; dann der bewegliche Stopfen (36) den ersten Verbindungskanal (34) sperrt, und die Verriegelungsanordnung (40) zur gleichen Zeit die Verbindungsstangenanordnung (20) sperrt; daher die Verbindungsstangenanordnung (20) unfähig ist, zu schwenken, und die angetriebene Einheit (37) und der bewegliche Stopfen (36) getrennt gehalten sind.

2. Modulares Kniegelenk nach Anspruch 1, worin die Verbindungsstangenanordnung (20) umfasst:
eine Hauptverbindungsstange (21), wobei die obere Verbindungseinheit (11) an der Hauptverbindungsstange (21) fest montiert ist, die Hauptverbindungsstange (21) einen Hauptschwenkschaft (210) umfasst, der zwischen einer Vorderseite der Hauptverbindungsstange (21) und einer Hinterseite der Hauptverbindungsstange (21) gelegen ist;
eine erste Verbindungsstange (23), die ein erstes oberes Ende und ein erstes unteres Ende umfasst, das erste obere Ende mit der Hauptverbindungsstange (21) verbunden ist, und das erste untere Ende an der Betätigungsanordnung (30) schwenkbar montiert ist; wobei ein Ende der angetriebenen Einheit (37) mit der ersten Verbindungsstange (23) verbunden ist;
eine zweite Verbindungsstange (24), die ein zweites oberes Ende und ein zweites unteres Ende umfasst, das zweite obere Ende mit dem Hauptschwenkschaft (210) der Hauptverbindungsstange (21) verbunden ist, und das zweite untere Ende an der Betätigungsanordnung (30) schwenkbar montiert ist; und
eine Hilfsverbindungsstange (22), wobei ein Ende der Hilfsverbindungsstange (22) an dem Hauptschwenkschaft (210) der Hauptverbindungsstange (21) schwenkbar montiert ist, und ein anderes Ende der Hilfsverbindungsstange (22) an dem ersten oberen Ende der ersten Verbindungsstange (23) schwenkbar montiert ist.

3. Modulares Kniegelenk nach Anspruch 2, worin die Verriegelungsanordnung (40) umfasst:
eine Verriegelungseinheit (41), wobei ein Ende der Verriegelungseinheit (41) mit der Hauptverbindungsstange (21) verbunden ist, und ein anderes Ende der Verriegelungseinheit (41) die erste Verbindungsstange (23) selektiv drückt;
worin, wenn die obere Verbindungseinheit (11) und die untere Verbindungseinheit (12) sich relativ zueinander drehen, die Verriegelungseinheit (41) die erste Verbindungsstange (23) drückt und dadurch die erste Verbindungsstange (23) daran hindert, sich relativ zur Hauptverbindungsstange (21) zu drehen.

4. Modulares Kniegelenk nach Anspruch 3, worin die Verriegelungsanordnung (40) umfasst:
eine elastische Einheit (42), wobei ein Ende der elastischen Einheit (42) mit der Hilfsverbindungsstange (22) verbunden ist, ein anderes Ende der elastischen Einheit (42) mit der Verriegelungseinheit (41) verbunden ist, und die elastische Einheit (42) dafür ausgelegt ist, die Verriegelungseinheit (41) anzutreiben, damit sie die erste Verbindungsstange (23) drückt.

5. Modulares Kniegelenk nach Anspruch 4, worin die Verriegelungsanordnung (40) umfasst:
eine Begrenzungseinheit (43), die an der Hauptverbindungsstange (21) montiert ist und zwischen der Verriegelungseinheit (41) und der ersten Verbindungsstange (23) gelegen ist.

6. Modulares Kniegelenk nach Anspruch 2, worin die Verbindungsstangenanordnung (20) umfasst:
eine dritte Verbindungsstange (25), die ein drittes oberes Ende und ein drittes unteres Ende umfasst, und das dritte obere Ende an der Hinterseite der Hauptverbindungsstange (21) geschwenkt ist.

7. Modulares Kniegelenk nach Anspruch 6, worin die Betätigungsanordnung (30) einen Stößelschaft (33) umfasst, der durch den zweiten Öllagerungshohlraum (32) montiert ist und mit dem dritten unteren Ende der dritten Verbindungsstange (25) verbunden ist.

8. Modulares Kniegelenk nach einem der Ansprüche 1 bis 7, worin die angetriebene Einheit (37) eine geneigte Ebene umfasst, die den beweglichen Stopfen (36) selektiv drückt.

9. Modulares Kniegelenk nach einem der Ansprüche 1 bis 7, worin die Betätigungsanordnung (30) eine angetriebene elastische Einheit (42) umfasst, die mit der angetriebenen Einheit (37) verbunden ist und dafür ausgelegt ist, die angetriebene Einheit (37) anzutreiben, um den beweglichen Stopfen (36) zu drücken.

## Revendications

1. Articulation de genou modulaire configurée pour relier une cuisse et une tige prothétique d'un utilisateur et **caractérisée en ce que** l'articulation de genou modulaire comprend :
une unité de connexion supérieure (11) configurée pour relier la cuisse de l'utilisateur ;
une unité de connexion inférieure (12) configurée pour relier la tige prothétique ;
un ensemble de bielle (20) situé entre l'unité de connexion supérieure (11) et l'unité de connexion inférieure (12) ;
un ensemble d'actionnement (30), l'unité de connexion inférieure (12) étant montée solidement sur l'ensemble d'actionnement (30); l'ensemble d'actionnement (30) comprenant :
une première cavité de stockage d'huile (31) ;
une seconde cavité de stockage d'huile (32) ;
un premier canal de connexion (34) en communication fluidique avec la première cavité de stockage d'huile (31) et la seconde cavité de stockage d'huile (32) ;
un second canal de connexion (35) en communication fluidique avec la première cavité de stockage d'huile (31) et la seconde cavité de stockage d'huile (32) ;
un obturateur mobile (36) bloquant sélectivement le premier canal de connexion (34) ;
**caractérisée en ce que** l'articulation de genou modulaire comprend en outre :
un ensemble de verrouillage (40) verrouillant sélectivement l'ensemble de bielle (20), par conséquent l'ensemble de bielle (20) est incapable de pivoter ; et
une unité entraînée (37), une extrémité de l'unité entraînée (37) reliée à l'ensemble de bielle (20), et une autre extrémité de l'unité entraînée (37) poussant sélectivement l'obturateur mobile (36) ;
dans lequel lorsque l'unité de connexion supérieure (11) et l'unité de connexion inférieure (12) tournent l'une par rapport à l'autre, l'unité entraînée (37) et l'obturateur mobile (36) sont séparés par l'ensemble de bielle (20) ; puis l'obturateur mobile (36) bloque le premier canal de connexion (34), et l'ensemble de verrouillage (40) verrouille l'ensemble de bielle (20) en même temps ; par conséquent, l'ensemble de bielle (20) est incapable de pivoter et l'unité entraînée (37) et l'obturateur mobile (36) restent séparés.

2. Articulation de genou modulaire selon la revendication 1, dans lequel l'ensemble de bielle (20) comprend :
une bielle principale (21), l'unité de connexion supérieure (11) étant montée solidement sur la bielle principale (21), la bielle principale (21) comprenant un arbre de pivot principal (210) situé entre un côté avant de la bielle principale (21) et un côté arrière de la bielle principale (21) ;
une première bielle (23) comprenant une première extrémité supérieure et une première extrémité inférieure, la première extrémité supérieure étant reliée à la bielle principale (21), et la première extrémité inférieure étant montée pivotante sur l'ensemble d'actionnement (30) ; une extrémité de l'unité entraînée (37) reliée à la première bielle (23) ;
une deuxième bielle (24) comprenant une deuxième extrémité supérieure et une deuxième extrémité inférieure, la deuxième extrémité supérieure étant reliée à l'arbre de pivot principal (210) de la bielle principale (21), et la deuxième extrémité inférieure étant montée pivotante sur l'ensemble d'actionnement (30) ; et
une bielle auxiliaire (22), une extrémité de la bielle auxiliaire (22) montée pivotante sur l'arbre de pivot principal (210) de la bielle principale (21), et une autre extrémité de la bielle auxiliaire (22) montée pivotante sur la première extrémité supérieure de la première bielle (23).

3. Articulation de genou modulaire selon la revendication 2, dans laquelle l'ensemble de verrouillage (40) comprend :
une unité de verrouillage (41), une extrémité de l'unité de verrouillage (41) reliée à la bielle principale (21), et une autre extrémité de l'unité de verrouillage (41) poussant sélectivement la première bielle (23) ;
dans laquelle lorsque l'unité de connexion supérieure (11) et l'unité de connexion inférieure (12) tournent l'une par rapport à l'autre, l'unité de verrouillage (41) pousse la première bielle (23) et empêche ainsi la première bielle (23) de tourner par rapport à la bielle principale (21).

4. Articulation de genou modulaire selon la revendication 3, dans laquelle l'ensemble de verrouillage (40) comprend :
une unité élastique (42), une extrémité de l'unité élastique (42) reliée à la bielle auxiliaire (22), une autre extrémité de l'unité élastique (42) reliée à l'unité de verrouillage (41), et l'unité élastique (42) configurée pour entraîner l'unité de verrouillage (41) pour pousser la première tige de connexion (23).

5. Articulation de genou modulaire selon la revendication 4, dans laquelle l'ensemble de verrouillage (40) comprend :
une unité de limitation (43) montée sur la bielle principale (21) et située entre l'unité de verrouillage (41) et la première bielle (23).

6. Articulation de genou modulaire selon la revendication 2, dans lequel l'ensemble de bielle (20) comprend :
une troisième bielle (25) comprenant une troisième extrémité supérieure et une troisième extrémité inférieure, et la troisième extrémité supérieure pivotée vers l'arrière de la bielle principale (21).

7. Articulation de genou modulaire selon la revendication 6, dans laquelle l'ensemble d'actionnement (30) comprend une tige de piston (33) montée à travers la seconde cavité de stockage d'huile (32) et reliée à la troisième extrémité inférieure de la troisième bielle (25).

8. Articulation de genou modulaire selon l'une quelconque des revendications 1 à 7, dans laquelle l'unité entraînée (37) comprend un plan incliné poussant sélectivement l'obturateur mobile (36).

9. Articulation de genou modulaire selon l'une quelconque des revendications 1 à 7, dans laquelle l'ensemble d'actionnement (30) comprend une unité élastique entraînée (42) reliée à l'unité entraînée (37) et configurée pour entraîner l'unité entraînée (37) pour pousser l'obturateur mobile (36).
